(19) Europäisches Patentamt
European Patent Office
Office européen des brevets

(11) Publication number: 0 155 252
A2

(12) # EUROPEAN PATENT APPLICATION

(21) Application number: 85850088.7

(22) Date of filing: 12.03.85

(51) Int. Cl.⁴: G 01 N 33/545

(30) Priority: 14.03.84 SE 8401437

(43) Date of publication of application: 18.09.85
Bulletin 85/38

(84) Designated Contracting States: BE DE FR GB IT NL SE

(71) Applicant: BIO-LINK ACTIVATED POLYMERS AB, Övre Slottsgatan 26 G, S-75235 Uppsala (SE)

(72) Inventor: Göthe, Sven, Allhelgonavägen 10, S-116 58 Stockholm (SE)
Inventor: Hult, Anders, Anbudsvägen 3C, S-183 44 Täby (SE)
Inventor: Johansson, Gunnar, Valloxvägen 45, S-741 00 Knivsta (SE)
Inventor: Larsson, Per, Lejdarvägen 65, S-149 00 Nynäshamm (SE)

(74) Representative: Larfeldt, Helene et al, Axel Ehrners Patentbyrå AB P.O. Box 5342, S-102 46 Stockholm (SE)

(54) **Immunoreactive solid phase.**

(57) A process for activating a solid polymer surface by radiation grafting of vinyl monomers having at least one functional group which can be bound to biologically active molecules, as well as a process for preparing an immunoreactive solid phase wherein said biologically active molecules are covalently bound to said functional groups on the polymer surface. The processes are characterized in that the polymer surface is grafted in solution in a chain transfer reducing solvent at a monomer concentration below 3% by weight when the vinyl monomer is 1-mono-or 1,1-disubstituted, whereby a very thin layer is obtained.

The invention also refers to the activated solid phase, obtained by the activating process, for immobilizing biologically active molecules and an immunoreactive solid phase comprising said molecules.

The immunoreactive solid phase to which an antigen or an antibody has been covalently bound can be used for quantitative or qualitative determination of antigen or antibody in an immunoassay.

EP 0 155 252 A2

## IMMUNOREACTIVE SOLID PHASE

The present invention refers to a process for activating a solid polymer surface by radiation grafting and the activated solid phase obtained. The invention also refers to a process for preparing an immunoreactive solid phase wherein biologically active molecules are covalently bound to the activated solid phase, as well as the immunoreactive solid phase obtained.

Specifically the invention also refers to the use of said immunoreactive solid phase in immunoassay.

In order to adapt polymeric materials to given functions and environmental demands it is often desirable to be able to modify the surface properties. It might for instance be a question of changing a hydrofobic surface into a hydrofilic, preparing substances with biocompatible characteristics or selective membranes. Another important application is surface binding of chemically or biologically active molecules, which are in this way immobilized and can act as reagents or catalysts.

By means of antibodies directed against a special antigen very sensitive assay methods can be developed, so called immunoassays (IA). If any included reagent is labelled with an isotope one speaks of radio immuno assay (RIA) and if a reagent is labelled with an enzyme, correspondingly of enzyme immuno assay (EIA). If larger amounts of antibody are used preparative systems might instead be developed.

A general problem with IA is to obtain a convenient separation of labelled reacted reagent from free labelled reagent. Separations and/or adsorptions based on differences in size or charge have been used. A better way is to immobilize either the antibody or the antigen. If the reagent is bound

to a solid phase already when added to the reaction mixture one speaks about "solid phase" technique. It is also possible to immobilize the reagent in a separate step by adding a secondary antibody directed against the antibody taking part of the reaction, the so called "double antibody" technique.

There are many advantages with the solid phase methods in IA which have been used to a great extent. As solid phase has been used cellulose in the form of beads or paper discs and plastic surfaces as PVC, polystyrene and polymethacrylates.

The ideal solid phase should be able to bind irreversibly large amounts of the reagent per surface unit. No reagent taking part in the reaction should in any significant amount bind unspecifically to the solid phase (background). In order to bind the reagent to the surface expensive chemicals should not be required, the process should be simple, it should be mild to the actual reagents (antibody and antigen), and the active groups on the surface should be stable in order to make it possible to store the material and bind the reagent at another time.

There is no presently available solid phase which fulfills these criterions. The solid phase described in this invention fulfills all the criterions.

Cellulose particles, Sepharose particles or slips of paper, which have been activated with cyanogen bromide (Axén et al., Nature 214:1302, 1967) can be used as a solid phase. This solid phase certainly has a high capacity for irreversible linking of protein reagent, but the background is so high that it can only be used for certain applications. Thus, it is well adapted for assay of IgE and antibodies of IgE-type against different antigens but not as well or not at all for determining IgG-antibodies. Another practical disadvantage

is that the solid phase to which the reagent is coupled by the experimentator has to be placed in a test tube or the like before the assay. In addition a special equipment is required for the washing steps always being a part of an IA. This type of solid phase is commercially available (Pharmacia Diagnostics) and is constantly used for analyzing proteins in general as well as specific antibodies of IgE-type.

Another type of solid phase mainly used in EIA is PVC and polystyrene to which a reagent has been passively adsorbed. This means that the reagents are reversibly bound to the plastic surface by means of unspecified forces. The degree of binding depends among other things on the charge of the reagents and in certain systems a strong binding of polysaccharides is obtained. Different proteins show different binding properties, in our investigations low values were obtained for human serum albumin, intermediary values for human IgG and moderately high values for human IgM. The advantage of this system is that the background is low, which makes it possible to determine IgG antibodies against different antigens. Plastic cuvettes in the shape of so called microtiter plates are commercially available, where the optical properties of the plastic make it possible to perform the photometric reading of the colour, the formation of which is catalyzed by the enzyme, directly in the reaction cuvette without any need of transferring into a separate glass cuvette. The washing procedures are also simpler than in those cases, as mentioned above about CNBr activated cellulose, as the reagents are bound directly to the inside of the tube or the "cuvette" in which the EIA reaction is performed. The drawbacks are that the binding is reversible, not specific for proteins, that the degree of binding varies with the charge of the proteins, that large variations can appear owing to the quality of the plastic surface of the tubes or microtiter plates from one manufacturing batch to another and that compared to for instance the CNBr method an unsufficient amount of reagent can be adhered to a given area.

SE 400 565 describes another solid phase technique, based on the formation of a hydrogel on a polymeric film to which biologically active molecules can be covalently bound. The method described can however not be used to graft for instance tubes or microtiter plates, as the liquid phase is gelling to a plug hard to remove. In using a solid phase coated or grafted with thick hydrogels the background will in addition be unacceptably high, which strongly reduces the sensitivity and the specificity of the test.

By means of the present invention chemical groups can be grafted on the surface of plastic as a thin film (<100 nm) for the preparation of an activated solid phase, which can be utilized for covalent linking of proteins. This surface specific method has a capacity per surface area that can be made independent of the charge of the proteins, that is irreversible, and that does not affect the optical properties of the plastic surface, why the "cuvettes" in the microtiter plate in the same way as in passive adsorption, can be read directly in a photometer. The plastic surface to which active groups have been added is stable and can be stored for a long time, for several months at room temperature, which means that in a technical scale production one is not dependent on that the activation of the plastic surface and the coupling of protein takes place continuously. This must be regarded to be a great advantage.

By the process of the invention a solid polymer surface is activated by radiation grafting of vinyl monomers having at least one functional group, which can be bound to biologically active moleculès.

In order to bind new molecules to a polymeric surface this has to have some kind of "active centra" comprising functional groups such as for instance OH, $NH_2$, COOH, CHO, NCO, SCO. These centra advantageously can be prepared by surface grafting of the polymer by radiation.

In many applications of polymer materials, the chemical and physical properties of the surfaces are as important as the properties exhibited by the bulk material. This realization has stimulated several groups to develop methods for chemical modification of the surface of polymers. The most common surface modification technique involves electron beam or γ-irradiation of the sample followed by free radical initiated, graft polymerization (A. Shapiro, "Radiation Chemistry of Polymers", Interscience Publishers, New York, 1962).

Surface modification via grafting reactions has been performed both in situ, by irradiation of the polymer surface in the presence of a reactive monomer, and by pre-irradiation followed by exposure to monomer in a subsequent step. The grafting reaction has, in turn, been carried out in two ways. Either the irradiated sample has been transferred in vacuo to a chamber in which the grafting reaction takes place or exposed to oxygen during or immediately after the irradiation. This latter process results in the formation of intermediate peroxides and hydroperoxides on the polymer surface which can be subsequently thermolyzed to form alkoxy radicals that function efficiently in the initiation of graft polymerization.

Radiation induced free radical graft polymerization has also been accomplished using UV light. Here, polymer samples were irradiated in a solution containing a vinyl monomer and a photosensitizer or were irradiated after the surface of the sample was saturated with such a solution. There are a few reports describing high energy radiation induced, ionic graft polymerization. Evidence for an ionic mechanism is generally the successful grafting of a monomeric species, such as vinyl ether, that is not prone to radical initiated polymerization.

Among polymers which are convenient to use in this connection could be mentioned polystyrene, polyethylene, poly-

propylene, poly-4-methylpentylene, polyvinyl chloride, polyester, polymethyl methacrylate.

A solution of the substance to be grafted on the surface is then irradiated with an adequate radiation dose under an inert atmosphere. The irradiation can be performed with radiation such as ultraviolet or ionizing radiation (nucleid, electronic, plasma). The temperature at the irradiation can be selected within a broad range, low temperatures being of advantage to prevent changes in optionally present biologically active substances intended to be immobilized at the surface of the object.

The process for activating a solid polymer surface of the invention is characterized in that the polymer surface is grafted in solution in a chain transfer reducing solvent at a monomer concentration below 3 % by weight when the vinyl monomer is 1-mono- or 1,1-disubstituted in order to prevent homopolymerization and an uncontrolled autocatalytic reaction.

The vinyl monomer to be grafted on the polymer surface can be described by the formula

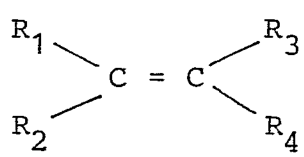

wherein at least one of $R_1$ - $R_4$ comprises a functional group, which can be covalently bound to a biologically active molecule. If $R_1$ and $R_2$ or $R_3$ and $R_4$ respectively are both hydrogen, the monomer is said to be 1-mono- or 1,1-disubstituted. If on the other side $R_1$ and $R_2$ or $R_3$ and $R_4$, respectively, are not both hydrogen the monomer is said to be 1,2-substituted.

Vinyl monomers which can be used in accordance with the invention are for instance crotonic acid, acrylic acid,

acrylic acid esters, acrylamide, bisacrylamide, methylol acrylamide, acrylated amines, acrylated epoxides, acrylated ethers, acrylated polyesters, acrylated polyurethanes, acrylated acrylates, acrylated silicons, acrylated silanes, acrylated phosphates and acrylated titanates, acrolein, phenyl substituted styrene derivatives as p-aminostyrene, tiglic acid, senecioic acid, angelic acid, cinnamic acid etc.

In order to get useful surfaces for high resolution affinity chromatography it is essential to be able to control the surface modification reaction. If an uncontrolled reaction takes place the data will be more or less useless. This is often the case if an unappropriate solvent or a too high monomer concentration (>10 % w/w) is choosen. It is very easy to start an autocatalytic polymerization reaction (the Tromsdorff effect) where the rate cannot be controlled. According to the invention the grafting can be controlled by using a very low monomer concentration (e.g. about 2 % w/w) when the vinyl monomer is 1-mono- or 1,1-disubstituted in combination with an appropriate solvent or by using 1,2-disubstituted vinylmonomers like crotonic acid. These monomers only form di- or trimeres on the surface and cannot undergo homopolymerization. Crotonic acid grafting according to the invention will always give a very well defined graft layer, due to the probable fact that further free radical reactions are inhibited by the molecular configuration of the monomer molecules (e.g. steric hindrance) and thus, further polymerization will not occur. The graft layer obtained will be very close to a monomolecular one. This is considered to be a novel and important advantage. This can be discussed in terms of "solid phase topography" and it is believed that the solid phases commonly used today, cellulose discs, agarose gels, Sepharose and Sephadex particles etc. are inferior in that aspect. In many instances the porous nature of these solid phases demonstrate a complex array of undesirable unspecific affinity reactions towards the reagent components in a given immunoassay system.

That may be due to pure mechanical trapping of reagent molecules but also depending on cooperating hydrofobic, polar - non-polar or local charge effects in a highly 3-dimensional solid phase. With the solid phases prepared according to our invention those side effects are minimized in that their topography may be described as close to 2-dimensional. In preparing solid phases with easy polymerizable vinyl monomers it is thus important to minimize the degree of further polymerization in order to get a thin, definable graft layer. In that aspect it is essential to keep the monomer concentration low in the grafting solution. Another alternative would be to perform the grafting process with the monomer present in a gaseous state. Further polymerization induced and generated by free radical formation can also be inhibited or limited by the presence of hydrated protons.
The solvent for the graft substance which during the irradiation shall cover the surface to be grafted has been defined as a chain transfer reducing solvent which in addition must not be detrimental to the plastic material. By chain transfer reducing solvent is here intended a solvent which when irradiated forms radicals not being able to initiate polymerization. As examples of suitable solvents can be mentioned methanol, pyridine, water and mixtures of methanol/ water.

The shape of the polymer is arbitrary and its size can vary within broad limits. It might for instance have a regular geometric shape which is for instance the case with discs, rods, reagent plates, sheets, bands, spheres etc. but it can also have an irregular shape.

As mentioned above a general advantage of our invention is that as solid phase substrate can be used commercially available surfaces, for instance test tubes and microtiter plates which are of interest for IA. By this a flexible system is obtained for those who want to prepare reagents for IA as there are many different microtiter plates and

tubes available. In addition there is of course a possibility to utilize quite different physical shapes of plastic for instance capillaries, the internal surface of which could be lined by active groups, to which reagents are linked covalently or small sticks, which are treated correspondingly and at the performance of the IA test are dipped into a biological specimen, for instance urine.

In the process for preparing an immunoreactive solid phase of the invention, biologically active molecules are bound to the functional groups on the activated solid phase.

By the use of coupling methods known per se said active molecules can be linked covalently to the plastic surface. As examples of coupling methods can be mentioned reaction with glutaraldehyde, cyanogen bromide, hydrazine, bisepoxyranes, divinyl sulfone, carbonyl diimidazole and other reagents being stated in the handbook from IBF LKB, "Practical guide for use in affinity chromatography and related techniques", Réactifs IBF - Société Chimiques Pointet-Girard, FR, 1983.

The biologically active molecules can, however, also only be adsorbed to the plastic surface wherein a so-called secondary binding or ionic binding is used. By secondary binding is here intended a binding which has been achieved by means of dispersive forces, polar forces or hydrogen bondings.

Biologically active molecules are for instance enzymes, hormones, antibiotics, pharmaceuticals, haptens, antigens and antibodies.

The immunoreactive solid phase of the invention comprising a polymer substrate with surface grafted functional groups to which biologically active molecules have been covalently linked can be used for quantitative or qualitative determination of antigen or antibody in an immunoassay.

Immunoreactive solid phases prepared according to our invention have, when used in various immunoassay systems, proven to be superior to many of the commonly used commercial ones. The field of application is very wide. Coupling of a biologically active component which functions as an antigen in a specific antibody - antigen equilibrium reaction, could thus be used for determination of absolute or relative levels of specific antibodies to the solid phase attached antigen. The widespread method of RAST (Paper Radio Allergo Sorbent Test, Pharmacia, Uppsala, Sweden) for the determination of IgE specific antibodies to common allergens, uses cellulose discs with covalently linked allergen. This method is suitable for detection of specific antibodies of the IgE but not of the IgG class, due to high unspecific binding in the latter case. After preparing microtiter plates with covalently linked timothy allergen in accordance with the invention seasonal variations of timothy specific IgG antibodies in allergic children could be studied in an EIA (L. Nordvall et al to be published). Such a study was impossible to perform with commercially available RAST timothy discs. Some comparisons can be made between immunoassays performed with solide phases prepared according to our invention and commercially used ones.

Determination of total IgE levels in human serum:
Commercial method PRIST (Paper Radio Immuno Sorbent Test, Pharmacia) a radio immunoassay performed on paper discs.
Lower detection limit: 120 pg IgE
Upper - " - -"- : 5-10 ng IgE
EIA in polystyrene microtiter plates of the invention (ex. 8)
Lower detection limit: 10 pg IgE
Upper -"- -"- : 5-10 ng IgE
Determination of timothy allergen specific IgE levels in human serum:
Commercial method RAST (Pharmacia) a radioimmunoassay performed on paper discs.
Lower detection limit: 50 pg IgE
EIA in polystyrene microtiter plates of the invention (ex10)
Lower detection limit: 1 pg IgE

On comparison with routinely used EIA-methods, based on passive adsorption, for determination of specific IgG-levels against different antigens it has consequently been found that immunoreactive solid phases prepared according to the invention gave an average of a 50 - 100 times higher resolution.

On outlining the general immunoassay applicabilities and advantages of our invention, an overall general preparation scheme could serve as a useful example.

Stage I: Preparation of an activated solid phase; the grafting process

The choice of a suitable polymer substrate is not in any way limited but the apparent major advantage is that commercially available plastic materials will do perfectly well. Examples of cheap suitable materials are PVC, and/or polystyrene test tubes, microtiter or tissue plates, and Petri dishes. Depending on the monomer which is to be grafted on to the polymeric substrate there are two different strategies to be aware of:

a) if the vinyl monomer is 1,2-disubstituted, e.g. crotonic, senecioic and tiglic acid, a 10 % by weight monomer solution should be used;

b) if the vinyl monomer is 1-mono- or 1,1-disubstituted, e.g. acrylic acid, a solution which contains the monomer in less than 3 % by weight is essential for reproducible results and should be used. Methanol-water in a 1:1 (by weight) proportion has throughout been used as a solvent. The reasons behind the choice are that the polymer in contrast to the graftmonomer substrate must be practically non-soluble in the solvent and it must function as an effective electron capturer. The presence of oxygen inhibits the grafting reaction and makes it important to use carefully deaireated monomer solutions. Fifteen minutes under nitrogen will bring the oxygen concentration down from 1 mM to a sufficient 10 µM. Thus stage I comprises the use

of for instance 10 % (by weight) crotonic acid in a MeOH - $H_2O$ (1:1 by weight) solutions substrate or a 2.5 % (by weight) of for instance acryl amide in the same solvent, preferably added with up to 20 mM nitric acid, on a polymeric substrate it also comprises the use of high energetic radiation for the induction of the free radical reaction, which under these conditions will occur mainly on the surface of the polymeric substrate. Optimum conditions for polystyrene grafting in a gammaradiation source (e.g. $^{60}Co$) are a total 2.5 - 3 MRad delivery at a rate of approximately 0.25 MRad/h. However, one of the crucial differences and novelties of the invention is the use of low graft monomer concentrations when highly polymerizable vinyl compounds are to be grafted. On using previously described concentrations (e.g. 10-20 by weight) the result will be not an ultrathin, well-defined grafting layer, but a thick, extensively polymerized gel plug. Utilizing such a reactive solid phase for immunoassay purposes would in many instances bring back the general background problems, previously discussed. It should also be stressed that a reactive solid phase prepared in that way cannot be used in a colorimetric EIA test where one wish to use for instance the individual wells in a microtiter plate as cuvettes, as the optical properties of the polymeric substrate will be destroyed.

Stage II: Reacting an activated solid phase; requirements for covalent binding of immunoassay solid phase reagents (e.g. biologically active molecules)

There are today a lot of commonly used and known methods for covalent coupling. The functional groups of the grafted surface and the chemical properties of the biologically active component, which is to be covalently linked, will govern the choice of a suitable condensating agent. We have mainly been working with solid phase carboxyl and amino groups and protein crosslinking by means of water soluble carbodiimides or glutaraldehyde. The use of carbodiimide crosslinking is somewhat limited by the requirement of

pure or highly refined biologically active components, because of the advantage to work at their isoelectric point, in order to achieve optimal binding conditions. Glutaraldehyde permits working in a wider pH range under coupling conditions and might in that aspect be considered as a more generally useful and among other things also a much cheaper condensating agent.

The solid phase of the invention can also be utilized in other applications than IA.

White blood cells and tumor cells can be identified and characterized by means of cell surface markers. This test can be performed by mixing for instance fresh sheep blood cells with a suspension of human white blood cells. Certain types of white blood cells will then link several sheep blood cells forming a so-called rosette. By means of particles of plastic or copolymers to the surface of which specific substances have been bound, sheep blood cells can be substituted and also other types of reagents be developed, being able to identify other types of white blood cells. By means of our invention polystyrene particles having active chemical groups on the surface can be used.

By using more material in order to get a greater amount of reagents available the solid phase technology can also be used for the purpose of separation.

Our invention could also be used in other medical connections where tissue compatible surfaces optionally having specific reagents covalently linked are desirable.

The invention can also be used in other connections when proteins or of course carbohydrates, if desired, are to be linked specifically to active groups on a polymer surface.

The invention will be illustrated by the following examples.

GRAFTING PROCESS

To a solution of methanol-$H_2O$ (1:1 by weight) is added 10% by weight crotonic acid. This solution was bubbled with nitrogen gas ($N_2$).

This mixture was added to a microtiter plate (e.g. PS) or tube in an inert atmosphere. The tube and/or microtiter plate is sealed if necessary to maintain the inert atmosphere.

This specimen is irradiated with a radiation dosis of 0.1-5 Mrad dependent on the conditions such as the plastic material, solvent composition, monomer. For instance a microtiter plate of PS, the solution mentioned above and a radiation dosis of about 3 Mrad. When ionizing radiation from $^{60}Co$ of 0.25 Mrad/h is used the specimen will be irradiated for 10-12 hours. The irradiated specimen is washed with distilled $H_2O$ before being ready for use.

The same process can be utilized for grafting with acrylic acid, but the monomer concentration is then lower, 2.5 % by weight, to avoid gelling. Grafting of for instance acrylamide, 2-hydroxy ethyl acrylate and 2-hydroxy ethyl methacrylate can be performed in a similar manner but in order to limit extensive polymerization the monomers must be kept at low concentrations (e.g. < 3 % by weight).

BINDING CAPACITY

Example 1

Object: The purpose of the following example was to quantify the protein binding capacity of a grafted polyvinyl chloride (PVC) surface as a function of available carboxylic groups on the surface which can be carbodiimide coupled. A comparative test was made between a grafted polycarboxylic acid (acrylic acid) and a grafted monocarboxylic acid (crotonic acid).

Performance: PVC microtiter plates (flat bottomed 280 µl, Flow Lab.) were grafted with crotonic acid and acrylic acid respectively (as to the process reference is made to the separate description). After the grafting process

(3 h, 0.31 Mrad/h, $^{60}Co$, $N_2$ atmosphere) the plates were washed with distilled water and allowed to dry. The plates were then kept in plastics bags at +20°C until they were used. 400 µg human gammaglobulin (HGG, Kabi) in 1 ml phosphate buffered physiological saline was labelled with 0.5 mCi (18.5 MBq) $Na^{125}I$ (Amersham, England) by means of Iodo Beads (Pierce Chem. Com., USA) according to the directions of the producer. The labelled protein was transferred into a 3 mM phosphate buffer (pH 6.3), called coupling buffer, by passage over a Sephadex G25 M column (PD10, Pharmacia) in accordance with the directions of the producer, equilibrated with coupling buffer. The protein concentration was adjusted to 100 µg/ml. 1 ml labelled HGG was mixed with 1 ml unlabelled HGG (1.9 mg/ml in coupling buffer). This gave a protein concentration of 1000 µg/ml. A serial dilution in coupling buffer was also made to give the following concentrations: 500, 200, 100 and 10 µg/ml.

To every well in two PVC plates, grafted with crotonic acid and acrylic acid respectively was added 50 µl of different protein concentrations. Then 50 µl EDAC (1-ethyl-3-[3-dimethylaminopropyl]-carbodiimide-HCl, mw 191.7 g/mol (Bio-Rad) dissolved in coupling buffer was added. The concentration of EDAC was 100 µg/ml. The plates were sealed with self-adherent plastic film and placed in a shaker at +4°C over night (incubation time ~18 h). The following day the incubation solution was aspirated and the plates were washed ten times with phosphate buffered saline (0.15 M pH 7.4), containing 0.1 % Tween 20 (Kebo). Bound radioactivity was then measured in a γ-counter (Multigamma, LKB).

Result: By relating measured cpm values to added total activity and known protein concentration the bonded protein could be quantified. The linking capacity was plotted as a function where log (nanogram) linked protein = f (log nanogram) added protein). Acrylic acid turned out to give a higher linking capacity compared to crotonic acid. The

maximum values were 7 µg and 5.5 µg/well respectively. At added protein concentrations below 20 µg/ml both acids showed a linking degree close to 100 %.

Example 2

Object: The purpose of the following example was to quantify the protein binding capacity of an acrylic acid grafted PVC surface and to compare the capacity of the grafted surface with the ability of an ungrafted PVC surface to bind protein by passive adsorption.

Performance: The grafting process and the subsequent treatment were performed in accordance with Example 1. For the test a PVC microtiter plate grafted with acrylic acid in this way was used and as a comparison an untreated plate. Bovine serum albumin (BSA, Cohn fr V.A. Johnsson & Co.), was labelled with $Na^{125}I$ (amounts and process according to Example 1). To the acrylic acid grafted plate was added BSA and coupling reagent according to Example 1. Before the addition of protein to the untreated PVC plate, the protein was transferred into an adsorption promoting buffer. This buffer consisted of 0.1 M sodium carbonate of pH 9.6. The transfer was done by passage over a PD 10 column, equilibrated with said carbonate buffer. To the untreated plate this protein was then added in amounts equivalent with those which were added to the grafted plate. No coupling reagent (EDAC) was added to the untreated plate. The total added volume per well (100 µl) was however the same. Incubation, washings, treatment and radioactivity measurements were performed in accordance with Example 1.

Result: The quantification was made in accordance with Example 1. The maximum binding capacity of the acrylic acid grafted PVC plate was 6.5 µg/well). The corresponding value for the untreated PVC plate having adsorption bound protein was 0.1 µg/well.

Example 3

Object: In accordance with Examples 1 and 2 but comparing the coupling capacity of a crotonic acid grafted PVC surface and the adsorption capacity of a polystyrene (PS) surface.

Performance: Grafting and subsequent treatment were performed in accordance with Example 1. For the adsorption comparison loose polystyrene wells (Removawell, Dynatech) were used. BSA was labelled with $Na^{125}I$ in accordance with Examples 1 and 2. For the adsorption to PS the protein was transferred into carbonate buffer in accordance with Example 2. Additions, incubation, washings, treatment and radioactivity measurements were made in accordance with Examples 1 and 2.

Result: The quantification was made in accordance with Example 1. The maximum binding capacity of the crotonic acid grafted PVC plate was 5 µg/well. For the untreated PS wells having adsorption bonded protein the corresponding value was 0.1 µg/well.

Example 4

Object: Demonstration of the protein binding capacity of an acrylamide grafted polystyrene surface after glutaraldehyde activation.

Performance: Acrylamide (AAM) was grafted on polystyrene tubes (Nunc Star Tubes) according to the previously described grafting process. The grafted surfaces were activated overnight at +37°C with a 25 % by volume aqueous glutaraldehyde solution at pH 7.6. After the activation the tubes were extensively washed with distilled water. Different amounts of a $Na^{125}I$ labelled rabbit immunoglobulin were added to the tubes in a total volume of 200 µl. The protein was dissolved in a 0.5 M phosphate buffer at pH 7.6. After incubation overnight on a tube shaker at a tempera-

ture of +4$^{\circ}$C the tubes were extensively washed with the washing buffer previously described. The tubes were then counted in a γ-counter and then washed again with a 10 % solution by volume of the detergent Decon 90® in order to elucidate the character of the protein binding. The tubes were then again counted in a γ-counter.

Result: Glutaraldehyde activated AAM tubes could bind up to 6 µg of rabbit immunoglobulin/tube. Of the added protein 90-95 % could be shown to be covalently linked as it could not be removed by the Decon 90® detergent.

Example 5

Object: The purpose of the following test was to optimize the EDAC concentration related to available carboxylic groups on a PVC surface. The carbodiimide should be added stoechiometrically in an excess of 10-100 times. A comparison was made between the ability of acrylic acid and crotonic acid grafted PVC plates respectively to bind human gammaglobulin (HGG) of various concentrations employing EDAC.

Performance: The grafting process and the subsequent treatment were performed in accordance with Example 1. HGG was labelled with $Na^{125}I$, treated and added in accordance with Example 1.

EDAC was added at a concentration of 10, 100 and 1000 µg/ml. Incubation, washings, treatment and radioacitivity measurements were made in accordance with Example 1.

Result: The quantification was made in accordance with example 1. Acrylic acid: In adding 50 µg HGG/well at EDAC concentrations of 1000 and 100 µg/ml respectively the higher concentration gave a 12.5 % higher binding capacity. At a EDAC concentration of 10 µg/ml quantitative additions lower than those described for passively adsorbing wells with BSA in accordance with examples 2 and 3 were obtained.

Crotonic acid: The binding capacity when adding 5 µg HGG/well at a EDAC concentration of 1000 and 100 µg/ml respectively was identical (1.1 ug) and in turn half of the corresponding value obtained for the acrylic acid. The EDAC concentration of 10 µg/ml gave a very low addition.

Example 6

Object: Passive adsorption to a plastic surface can be increased by grafting of a suitable group. The interactions between for instance a protein in solution and such a plastic surface can then be increased both as regards hydrophobic, hydrophilic and charge interaction. The purpose of the following example was to show that a PVC plate grafted with crotonic acid could passively adsorb a larger amount of protein than a corresponding untreated PVC plate and untreated loose polystyrene (PS) wells respectively.

Performance: The grafting process and subsequent treatment were performed in accordance with example 1. Human gammaglobulin (HGG) was labelled with $Na^{125}I$ and treated as in example 1. HGG was in addition transferred into carbonate buffer in accordance with the description in example 2.

To the crotonic acid grafted PVC plate was added 50 µl of the protein solution in concentrations according to example 1. Then 50 µl 3mM phosphate buffer of pH 6.3 was added. To the untreated PVC plate and the untreated PS wells respectively were added equivalent amounts and volumes of the protein, transferred into carbonate buffer, and then another 50 µl

carbonate buffer was added. Incubation, washings, treatment and radioactivity measurements were performed in accordance with example 1.

Result: The quantification was made in accordance with example 1.
Crotonic acid grafted PVC plate: The maximum binding capacity was 1.5 µg HGG/well.
Untreated PVC plate: The maximum binding capacity was 0.3 µg/well.
Untreated PS wells: The maximum binding capacity was 0.8 µg/well.

CHARACTERIZATION OF TYPE OF BINDING

Example 7

Object: The purpose of the following test was to distinguish between covalently bound (carbodiimide coupled) and passively adsorbed (without any coupling reagent) protein to a crotonic acid grafted polystyrene surface. The test also showed the degree of the covalent binding to the grafted surface.

Performance: A polystyrene (PS) microtiter plate (Dynatech, M 29 AR) was grafted with crotonic acid (reference is made to a separate description). After the grafting process (10 h, 0.3 MRad/h, $^{60}Co$, $N_2$ atmosphere) the plate was washed with distilled water and allowed to air dry. The plate was then sealed with self adhesive plastic film and kept at +20°C until used. 400 µg rabbit gammaglobulin was labelled with $Na^{125}I$ and treated in accordance with example 1. The protein was diluted with coupling buffer to a concentration of 100 µg/ml. To four wells in the plate were added 50 µl (5 µg protein) each of this solution. To two wells were then added 50 µl EDAC (1000 µg/ml in coupling buffer) each, and to the remaining two only 50 µl coupling buffer. The plate was then incubated in accordance with example 1. A duplicate of 50 µl labelled protein (5 µg protein) was measured in a γ-counter in order to obtain the total added activity. On the following day

50 $\mu l$ incubation solution was aspirated from each of the four wells.

The four specimens were measured in a $\gamma$-counter and the cpm values obtained for each specimen multiplied with 2 in order to obtain the remaining not linked activity. The mean value of cpm for each duplicate was then subtracted from the mean value of the added total activity. The values obtained were then regarded as a measure of bound activity (BA). The plate was then washed in accordance with example 1. After this washing 200 $\mu l$ 10 % Decon 90 (laboratory detergent, Decon Labs Ltd) was added to each of the four wells. The plate was placed in a shaker at ambient temperature for the night. The following day 100 $\mu l$ specimen was aspirated from each of the four wells. The four specimens were measured in a $\gamma$-counter and the obtained cpm values for each specimen was multiplied with 2 in order to get the washed away adsorbed activity (AA). The mean value of cpm for each duplicate was then subtracted from the corresponding values for bound activity. The values obtained were then regarded as a measure of covalently bound activity (KBA) of the coupled wells and for firmly adsorbed activity for the uncoupled ("KBA"). This was summarized in the following formula in which cpm mean values are used for each duplicate: KBA = BA - (2 x AA).

Result: Wells coupled with carbodiimide reagent
Bound activity (BA): 60 % (3 $\mu g$ protein)
Covalently bound activity (KBA): 50 % (2.5 $\mu g$ protein)

Wells without carbodiimide reagent:
Bound activity (BA): 38 % (1.9 $\mu g$ protein)
Covalently bound activity ("KBA"): 4 % (0.2 $\mu g$ protein).

APPLICATIONS ON IMMUNOASSAY (IA)

Enzyme immunoassay (EIA)

Example 8

Object: The purpose of this example was to show that a crotonic acid grafted polystyrene surface by means of a carbodiimide

reagent could be used to chemically link an antibody, the immunoreactivity being maintained, in such a way that it could then be used in a quantitative immunoassay of "double sandwich" type. In this case an EIA for quantitative determination of human IgE is described.

Performance: A polystyrene (PS) plate (Dynatech, Micro-ELISA, M129B) was grafted with crotonic acid and subsequently treated in accordance with Example 7. A gammaglobulin fraction of a rabbit hyperimmun serum pool (rabbit anti-IgE) having a specific immunoreactivity against the epitopes $D_{\varepsilon 1}$ and $D_{\varepsilon 2}$ on the human myelom protein IgE (ND) (S.G.O. Johansson & H. Bennich, 1967) was used for the coupling to a solid phase. Rabbit anti-IgE was transferred into a coupling buffer by passage over a PD10 column in accordance with example 1. The protein concentration was adjusted to 40 µg/ml. 50 µl of this solution was added to each of the wells of the plate. To each well was then added 50 µl EDAC (1000 µg/ml in coupling buffer). The plate was then incubated in accordance with example 1. On the following day the plate was washed 3 times with a phosphate buffered 1.5 M saline of pH 7.2. This was followed by 3 washings with washing solution described in example 1. An affinity purified human myelom protein, IgE(SB), the concentration of which was determined by the PRIST method (Pharmacia) in accordance with the instructions of the producer, was used in different dilutions as a standard. The dilutions were made in a phosphate buffered, physiological saline of pH 7.4 with the addition of 0.2 % BSA, 0.1 % Tween 20 and 0.02 % sodium azide. (This buffer will from now be called the assay buffer (AB).) The standard dilutions in AB comprised the following IgE concentrations: 1000, 500, 100, 50, 25, 10, 5, 1, 0.5, 0.1, 0.05 and 0.01 ng/ml. 100 µl of each concentration was added in duplicate to the wells of the plate. As a blank was added AB, 100 µl in quadruplicate. Two standard curves of this type with blanks included were added to the plate. After each curve in addition 100 µl duplicates of a commercial PRIST standard, diluted 1/20 in AB were added. The plate was then

shaked for 30 s in a shaker, sealed by self adhesive plastic film and incubated for 1 h at 37$^{o}$C. The plate was then washed 3 times with the washing solution described in example 1. To each standard curve with blanks and specimens two different types of antibody-enzyme conjugate were added. One conjugate consisted of a affinity purified rabbit antibody having a $D_{\varepsilon 1}$ specificity, which had been glutaraldehyde conjugated with alcaline phosphatase (ALP, Sigma, type VII-T) in the proportions 1/3 (by weight). This was called rabbit anti-$D_{\varepsilon 1}$ ALP. The second conjugate was of the same type but having a $D_{\varepsilon 2}$ specificity. This was named rabbit anti-$D_{\varepsilon 2}$-ALP. Each conjugate was diluted about 2000 times in AB. To each standard curve, blanks and specimens included, 100 µl/well of rabbit anti-$D_{\varepsilon 1}$-ALP and rabbit anti-$D_{\varepsilon 2}$-ALP was added respectively. The plate was then shaked for about 30 s in a shaker, sealed by self adhesive plastic film and incubated at ambient temperature over night. On the following day the plate was washed 3 times with a washing solution according to example 1. To each well was then added 100 µl of a substrate solution, consisting of para-nitrophenyl phosphate (pNPP, Sigma 104-105, tablets of 5 mg) 1 mg/ml in 1 M diethanolamine-HCl, pH 9.8 with 0.5 mM MgCl and 0.02 % $NaN_3$. The plate was shaked for 30 s in a shaker. The enzyme catalyzed reaction was followed on a spectrophotometer intended for microtiter plates (SLT 210.1, Kontron AB), by reading the absorbance at the wavelength 405 nm and the reference wavelength 620 nm. The spectrophotometer was then set to zero for each blank. (The apparatus subtracts the mean value for the given blanks from the absorbance values of the specimens).

Absorbance values obtained after reaction for 70 minutes were used for curve fitting by means of a so-called spline function (Treville, Reinsch, Hayes, Wold, Marschner et al) the absorbance at 405 nm being plotted as a function of the logaritm of the concentration of IgE.

Result: Standard curve detected with rabbit anti-$D_{\varepsilon 1}$-ALP:

| IgE ng/ml | Absorbance 405 nm (± σ of the mean value) |
| --- | --- |
| 500 | 1.785 ± 0.007 |
| 100 | 1.134 ± 0.046 |
| 50 | 0.612 ± 0.020 |
| 25 | 0.382 ± 0.002 |
| 10 | 0.172 ± 0.003 |
| 5 | 0.095 ± 0.001 |
| 1 | 0.022 ± 0.002 |
| 0.5 | 0.009 ± 0.001 |

The subtracted blank was 2.7 % of the absorbance value of the highest standard point + blank.

The commercial PRIST control which according to the producer should contain 100 kU/l (1U ≈ 2.4 ng IgE/ml) = 240 ng IgE/ml was mathematically extrapolated from the standard curve to 220 ± 10 ng/ml.

Standard curve detected with rabbit anti-$D_{\varepsilon 2}$-ALP:

| IgE ng/ml | Absorbance 405 nm (± σ of the mean value) |
| --- | --- |
| 50 | 1.714 ± 0.012 |
| 25 | 1.224 ± 0.011 |
| 10 | 0.730 ± 0.011 |
| 5 | 0.377 ± 0.011 |
| 1 | 0.073 ± 0.006 |
| 0.5 | 0.043 ± 0.003 |
| 0.1 | 0.024 ± 0.002 |

The substracted blank was 2.8 % of the absorbance value of the highest standard point + blank.

PRIST control (240 ng/ml) was extrapolated to 220 ± 2 ng/ml.

Example 9

Object: In analogy with example 8 but with the purpose of detecting and quantifying antigen specific IgG antibodies in human sera by means of antigen linked to a crotonic acid grafted polystyrene surface. Comparison was made with a parallelly running EIA routine method for the same type of antibody determination, being based on passive adsorption of the antigen to a solid phase.

Performance: A PS plate was grafted and subsequently treated in accordance with examples 7 and 8. The plate was coupled with phospholipase A2 (PLA2 from honey bee venom, Sigma P-2509) 2 µg/well, incubated and then treated in accordance with example 7. An untreated PS plate was allowed to adsorb PLA2 100 µl/well, 20 µg/ml in 0.1 M carbonate buffer pH 9.6, over night at +4°C. On the following day the plate was washed 3 times with a washing solution described in example 1. To the first 8 wells of each plate was added 100 µl assay buffer (AB), to be used as blanks. Then 100 µl dilutions in AB of a standard serum pool (from immunized bee keepers) were then added in duplicate, having a known content of PLA2 specific IgG antibodies, to each plate. The known dilutions contained 25, 10, 5, 2.5, 1 and 0.5 EIA units (EE) respectively of specific antibodies. (100 EE is defined as a dilution 10 times of the serum pool).

In the same way dilutions of unknown specimens were added to each plate. The plates were placed for 30 seconds in a shaker, sealed and were then incubated for 3 h at ambient temperature. They were then washed 3 times in accordance with example 8. To each well in each plate was then added 100 µl of a rabbit anti-human IgG-ALP conjugate, diluted 1/2000 in buffer in accordance with example 8. Rabbit anti-human IgG (Fc$\gamma$-specific, Dakopatts AB, A 090) was glutaraldehyde conjugated to alkaline phosphatase (ALP) in accordance with example 8. The plates were then incubated at ambient temperature over night and treated subsequently in an analogous way as in example 8. The plates were read in a spectrophotometer after a reaction time of 30 min.

| Result: Standard (EE7ml) | Crotonic acid grafted PS Abs 405 nm (coupled PLA2) | Untreated PS Abs 405 nm (adsorb. PLA) |
|---|---|---|
| 25 | 1.957 ± 0.008 | 1.715 ± 0.061 |
| 10 | 1.773 ± 0.007 | 1.524 ± 0.012 |
| 5 | 1.572 ± 0.003 | 1.257 ± 0.022 |
| 2.5 | 1.292 ± 0.070 | 0.966 ± 0.029 |
| 1 | 0.728 ± 0.025 | 0.502 ± 0.012 |
| 0.5 | 0.391 ± 0.011 | 0.181 ± 0.003 |
| | mean values | |
| Unknown specimens | EE/ml | EE/ml |
| 1 | 44 | 29 |
| 2 | 8 | 8 |
| 3 | 11.5 | 10 |
| 4 | 12 | 11 |
| 5 | 2 | 2 |
| 6 | 9.5 | 11 |
| 7 | 1.5 | 3 |
| 8 | 2.5 | 3 |
| 9 | 9 | 8 |
| 10 neg control* | 1.5 | 2.5 |

*(<5 EE/ml)

| | | |
|---|---|---|
| Variation of specimen | ± 5 % | +10 % |
| (deviation from the mean value) | | |
| Blank in % of the highest absorbance value plus blank value | 2.1 % | 2.4 % |

Example 10

Object: By analogy with example 9 but with the purpose to detect and quantify antigen (allergen) specific IgE antibodies in human sera by means of antigen (allergen) linked to a crotonic acid grafted polystyrene surface. The example was also performed in order to demonstrate the extremely high sensitivity of the method.

Performance: A PS plate was grafted and subsequently treated in accordance with example 7 and 8. The plate was coupled with timothy extract (Pleum pratense, Spectralgen, Pharmacia, 100 000 BE/ml after reconstitution to 4.5 ml). 10 µg/well diluted in coupling buffer was incubated and treated in accordance with example 8. A patient having a known content of specific IgE anti-timothy antibodies (800 PRU; determined by means of Pharmacia's RAST as instructed by the producer) was used to make a standard curve. 1 PRU was determined to correspond to 2.4 ng specific IgE/ml serum. The following standard points were used: 10000, 1000, 500, 250, 100, 50, 25 and 10 pg/ml, diluted in AB. 100 µl in duplicate was added to the wells of the plate. As a blank was used a duplicate of solely AB. The plate was incubated and washed in accordance with example 9. A mixture 1/1 of the conjugates described in example 8, diluted 1/2000 in AB were in a volume of 100 µl added to each well. The plate was sealed and incubated at 37°C over night.

On the following day the plate was washed and the substrate solution added in accordance with examples 8 and 9. Incubation and reading were also performed in accordance with examples 8 and 9.

Result: Specific IgE anti-timothy

| Standard (pg/ml) | Abs 405 nm |
|---|---|
| 1000 | 1.629 ± 0.070 |
| 500 | 0.921 ± 0.008 |
| 250 | 0.488 ± 0.027 |
| 100 | 0.196 ± 0.012 |
| 50 | 0.100 ± 0.007 |
| 25 | 0.069 ± 0.001 |
| 10 | 0.027 ± 0.002 |

Blank in % of the highest absorbance value + blank value = 0.47 %

Example 11

Object: In analogy with example 8 but demonstrating grafting, activation and protein coupling of 2-hydroxy ethyl acrylate on polystyrene, which was then used in an enzyme immunoassay for quantifying human IgE.

Performance: 2-hydroxy ethyl acrylate was grafted on a polystyrene microtiter plate (Dynatech Micro-ELISA) according to the previously described grafting process. The grafted plate was activated overnight in the dark with benzoquinone 30 mg/ml in absolute ethanol at +37°C. The plate was then washed 3 times with ethanol and 3 times with distilled water. 70 µg sheep immunoglobulin with specificity against the Fc portion of human IgE in a 0.1 M carbonate buffer at pH 9.0 was added to each well in a 100 µl volume. The plate was then shaked overnight at +4°C. Further additions of reagents and treatments were then performed as previously described in example 8.

Result: Standard curve detected with rabbit anti-$D_{\varepsilon 2}$-ALP

| IgE ng/ml | Absorbance 405 nm |
|---|---|
| 24000 | 2.461 |
| 2400 | 2.378 |
| 240 | 2.217 |
| 120 | 2.177 |
| 48 | 1.836 |
| 18 | 1.467 |
| 6 | 0.862 |
| 2.4 | 0.425 |
| 1.2 | 0.236 |
| 0.12 | 0.079 |
| 0.012 | 0.047 |
| 0.0012 | 0.013 |
| IgE free serum diluent | 0.009 |

PRIST control (240 ng/ml) was extrapolated to 220 ng/ml.

RADIOIMMUNOASSAY (RIA)

Example 12

Object: By analogy with example 8 the purpose of this example was to show that a polystyrene surface being equipped with a group that could be coupled could also be used in a radioimmuno assay (RIA). The example has been chosen with crotonic acid grafted polystyrene tubes which can be coupled with carbodiimide and is demonstrating a model which could be used for quantitative determination of human IgE.

Performance: Polystyrene tubes (Vidal, 12x75 mm) were grafted with crotonic acid (reference is made to a separate description). The volume was 250 µl and the radiation doses 3 Mrad for about 10 h.

Result:

| IgE (kU/l) | % of Tot |
|---|---|
| 0.25 | 0.73 |
| 0.5 | 0.90 |
| 1 | 1.25 |
| 2.5 | 2.01 |
| 7.5 | 4.75 |
| 20 | 11.84 |
| 50 | 20.40 |
| 100 | 27.44 |
| 250 | 36.46 |
| 500 | 38.42 |
| 1000 | 41.26 |
| 10000 | 40.05 |
| 100000 | 46.70 |
| diluent | 0.53 |

Total activity (Tot) = 55837 cpm

Example 13

Object: In analogy with example 12 the purpose of this example was to show that acrylamide grafted and glutaraldehyde activated polystyrene tubes can be used in a radioimmunoassay. The model chosen could be used for quantitative determination of human IgE.

Performance: Polystyrene tubes (Nunc Maxisorb.) were grafted with acrylamide (reference is made to the separate description). The grafting volume was 200 µl and activation with glutaraldehyde was carried out as described previously. The tubes were coupled with 5 µg of a sheep immunoglobulin with specificity against the Fc-portion of human IgE.

Results:

| IgE (kU/l) | % of Tot |
|---|---|
| 0.5 | 1.55 |
| 2.5 | 5.81 |
| 7.5 | 9.36 |
| 100 | 50.60 |
| diluent | 0.34 |

Total added activity (Tot) = 57615 cpm.

## C L A I M S

1. Process for activating a solid polymer surface by radiation grafting of vinyl monomers having at least one functional group, which group can be bound to biologically active molecules, c h a r a c t e r i z e d in that the polymer surface is grafted in solution in a chain transfer reducing solvent at a monomer concentration below 3 % by weight when the vinyl monomer is 1-mono- or 1,1-disubstituted in order to prevent homopolymerization and an uncontrolled autocatalytic reaction.

2. Process according to claim 1, c h a r a c t e r i z e d in that the monomer concentration can be arbitrary selected if the vinyl monomer is 1,2-substituted.

3. Activated solid phase for immobilizing biologically active molecules, comprising a polymer substrate to which functional groups, which can be bound to the biologically active molecules, have been surface grafted, c h a r a c t e r i z e d in that the polymer surface has been radiation grafted in accordance with claim 1 in a thin layer.

4. Activated solid phase according to claim 3, c h a r a c t e r i z e d in that the polymer substrate is a shaped plastic, such as polyethylene, polypropylene, polystyrene, polyvinyl chloride, polyester or polymethylmethacrylate.

5. Process for preparing an immunoreactive solid phase, comprising radiation grafting of vinyl monomers having at least one functional group to a solid polymer surface, and subsequent covalent binding of biologically active molecules to the functional groups by means of condensating agents and methods known per se, c h a r a c t e r i z e d in that the polymer surface is grafted in solution

in a chain transfer reducing solvent at a monomer concentration below 3 % by weight when the vinyl monomer is 1-mono- or 1,1-disubstituted in order to prevent homopolymerization and an uncontrolled autocatalytic reaction.

6. Process according to claim 5, characterized in that an 1,2-substituted acrylic acid is radiation grafted to a polystyrene substrate, and a biologically active molecule is covalently bound by means of a carbodiimide condensating agent.

7. Immunoreactive solid phase comprising a polymer substrate with surface grafted functional groups to which biologically active molecules have been covalently bound, characterized in that the immunoreactive solid phase has been prepared by the process of claim 5.

8. Immunoreactive solid phase comprising a polymer substrate to which an antigen or an antibody has been bound, characterized in that the antigen or antibody is covalently bound to functional groups which have been radiation surface grafted to the polymer substrate.

9. Immunoreactive solid phase according to claim 8, characterized in that the polymer substrate is a polystyrene or PVC microtiter plate or tube, to the inner surface of which has been grafted a functional group -COOH -OH or $-NH_2$, to which an antigen or an antibody has been covalently bound by means of a carbodiimide and a glutaraldehyde condensating agent respectively.

10. Use of an immunoreactive solid phase comprising a polymer substrate with surface grafted functional groups to which an antigen or an antibody has been covalently bound for quantitative or qualitative determination of antigen or antibody in an immunoassay.

11. Use of an immunoreactive solid phase according to claim 10, comprising a polystyrene microtiter plate or tube to the inner surface of which has been grafted a functional group $-COOH$, $-OH$ or $-NH_2$, to which an antigen or an antibody has been covalently bound, for quantitative or qualitative determination of antigen or antibody in an enzyme immunoassay.

-----0-----